Europäisches Patentamt

(19) European Patent Office

Office européen des brevets

(11) Publication number: **0 010 636**
**A1**

# (12) EUROPEAN PATENT APPLICATION

(21) Application number: **79103779.9**

(22) Date of filing: **03.10.79**

(51) Int. Cl.³: **C 07 D 473/12**, A 23 F 5/20, A 23 F 3/36

(30) Priority: **06.11.78 US 957821**

(43) Date of publication of application: **14.05.80**
**Bulletin 80/10**

(84) Designated Contracting States: **CH DE FR GB IT NL**

(71) Applicant: **SOCIETE DES PRODUITS NESTLE S.A.,**
**Case postale 353, CH-1800 Vevey (CH)**

(72) Inventor: **Margolis, Geoffrey, 42, Chemin de la**
**Potteilaz, CH-1030 Bussigny (CH)**
Inventor: **Chiovini, Jacky, CH-1349 Daillens (CH)**

(74) Representative: **Ledzion, Andrzej, 55, avenue Nestlé,**
**CH-1800 Vevey (CH)**

(54) **Caffeine extraction.**

(57) In the extraction of caffeine from a caffeineladen supercritical carbon dioxide phase with water, the carbon dioxide phase has a density not exceeding 0.75 g/ml.

EP 0 010 636 A1

ACTORUM AG

0010636

## Caffeine extraction

This invention is concerned with the extraction of caffeine from mixtures of caffeine and supercritical carbon dioxide.

As described in British Patent Specification No. 1290117, supercritical carbon dioxide is a solvent for caffeine, and may hence be used for the decaffeination of tea or coffee. The term "supercritical" denotes that the carbon dioxide is at a temperature and pressure which are greater than the critical temperature (31.3°C) and pressure (73.8 bar). According to this Specification, the caffeine may be extracted from the carbon dioxide phase by contact with active charcoal.

German Patent Application No. 2638383 describes the decaffeination of aqueous coffee extracts with supercritical carbon dioxide, and proposes that water be used for extracting caffeine from caffeine-laden carbon dioxide.

It has now been found that caffeine may be rapidly and efficiently extracted with water from mixtures of caffeine and supercritical carbon dioxide if on contact with water the density of the supercritical carbon dioxide does not exceed 0.75 g/ml.

The present invention thus provides a process for extracting caffeine from caffeine-containing supercritical carbon dioxide which comprises contacting a caffeine-containing supercritical carbon dioxide phase having a density not exceeding 0.75 g/ml with water and separating an aqueous solution of caffeine from a carbon dioxide phase of reduced caffeine content. In the present context, "reduced" is also intended to mean zero, so that the carbon dioxide separated may be completely devoid of caffeine. Preferably, 30 to 50 parts by weight of carbon dioxide are contacted with 1 part by weight of water. Lower ratios are, of course, also possible, and in fact provide more efficient caffeine removal. However, as at the same time the volume of water to be handled is increased, the indicated range represents a practical comprise between the two extremes.

It will be appreciated that the density of the carbon dioxide phase may be adjusted to the desired value by varying its temperature and/or pressure provided, of course, that both the higher than the critical values given above. In practice, it is preferred to maintain a constant pressure and vary the temperature. Thus, the pressure may be in the range 74 - 450 bar, and the temperature between 32 and 130$^\circ$C. Usually, the water is heated to the desired temperature prior to contacting.

Contacting may be effected in a packed column or other piece of equipment providing for intimate fluid-fluid contact and separation of phases. Extraction is most conveniently carried out in continuous, countercurrent manner, the aqueous phase being separated for recovery of caffeine. The carbon dioxide may be recycled to a decaffeination operation, for example of the type described in our application entitled "Decaffeination process" of even date herewith.

The process of the present invention is particularly useful for the extraction of caffeine from caffeine-laden supercritical carbon dioxide phases produced in the decaffeination of green or roasted coffee beans, tea, and aqueous extracts of tea or of green or roasted coffee. It may, however, also be used for the extraction of caffeine from caffeine-containing supercritical carbon dioxide phases obtained from other sources.

The invention is illustrated by the following Examples, in which all parts, percentages and ratios are expressed on a weight basis.

### Example 1

Caffeine-laden supercritical carbon dioxide, resulting from the decaffeination of an aqueous extract of roasted coffee and having a density of 0.75 g/ml, is contacted batchwise with water. The ratio of carbon dioxide to water is 30:1. 50 % of the caffeine present are transferred to the water. The caffeine solution is processed for caffeine recovery, whereas the carbon dioxide is used for decaffeination of further batches of coffee extract.

For comparison purposes, the procedure was repeated except that the carbon dioxide had a density of 0.91 g/ml. 29 % of the caffeine are transferred to the water phase.

### Example 2

Caffeine-containing supercritical carbon dioxide from various sources was continuously contacted countercurrent to water in a packed column. The operating conditions and results are given in the Table, asterisked data identifying comparative experiments outside the scope of the present invention.

## T A B L E

| Source of $CO_2$ | Ratio $CO_2$:water | Carbon dioxide | | | % Caffeine transferred to water |
|---|---|---|---|---|---|
| | | Temp. ($^{o}$C) | Press. (bar) | Density (g/ml) | |
| Aqueous extract of green beans | 40:1 | 90 | 200 | 0.55 | 93 |
| | 40:1 | 90 | 185 | 0.50 | 95 |
| | 40:1 | 70 | 400 | 0.86* | 36 |
| | 40:1 | 70 | 600 | 0.95* | 20 |
| Tea extract | 40:1 | 60 | 140 | 0.58 | 93 |
| | 20:1 | 60 | 140 | 0.58 | 99 |
| Roasted coffee extract | 45:1 | 90 | 185 | 0.50 | 93 |
| | 40:1 | 70 | 250 | 0.75 | 63 |
| | 40:1 | 90 | 200 | 0.55 | 93 |
| | 40:1 | 70 | 400 | 0.86* | 36 |
| | 20:1 | 60 | 140 | 0.58 | 98 |

- 5 -

0010636

CLAIMS:

1. A process for extracting caffeine from caffeine-containing carbon dioxide which comprises contacting a caffeine-containing supercritical carbon dioxide phase having a density not exceeding 0.75 g/ml with water and separating an aqueous solution of caffeine from a carbon dioxide phase of reduced caffeine content.

2. A process according to claim 1 in which 30 to 50 parts by weight of carbon dioxide are contacted with 1 part by weight of water.

3. A process according to claim 1 or claim 2 in which the contacting is effected at a temperature of $32 - 130^{\circ}C$.

4. A process according to claim 1, 2 or 3 in which the contacting is effected at a pressure of 74 - 450 bars.

| DOCUMENTS CONSIDERED TO BE RELEVANT | | | CLASSIFICATION OF THE APPLICATION (Int. Cl. ³) |
|---|---|---|---|
| Category | Citation of document with indication, where appropriate, of relevant passages | Relevant to claim | |
| DX | <u>DE - A - 2 638 383</u> (D.E.J. INTER-NATIONAL RESEARCH) <br><br> * Claims 1,2,5,12-17; page 3, paragraph 5 - page 4, paragraph 1 * <br><br> -- | 1,3,4 | C 07 D 473/12 <br> A 23 F 5/20 <br> A 23 F 3/36 |
| X | <u>DE - A - 2 221 560</u> (STUDIENGESELL-SCHAFT KOHLE) <br><br> * Claim; page 2, paragraph 4 - page 3, paragraph 1; figures* <br><br> -- | 1,3,4 | |
| | | | TECHNICAL FIELDS SEARCHED (Int.Cl. ³) |
| PE | <u>GB - A - 2 014 425</u> (STUDIENGESELL-SCHAFT KOHLE) <br><br> * Claims 1,4,5; page 2, lines 35-46; example 1 * <br><br> ---- | 1,3,4 | C 07 D 473/12 <br> A 23 F 5/20 <br> A 23 F 5/22 <br> A 23 F 3/36 <br> A 23 F 3/38 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| | | | |
|---|---|---|---|
| The present search report has been drawn up for all claims | | | |
| Place of search | Date of completion of the search | Examiner | |
| The Hague | 13-02-1980 | DESMEDT | |

EPO Form 1503.1   06.78